# EUROPEAN PATENT APPLICATION

(11) **EP 0 677 300 A1**
(43) Date of publication of application: **18.10.1995**
(21) Application number: 95200874.6
(22) Date of filing: 06.04.1995
(51) Int. Cl.: A61M 39/18, A61M 1/28

(54) **Bag supply and discharge system for peritoneal dialysis**

(30) Priority: 12.04.1994 IT MI940680
(71) Applicant: PIERREL OSPEDALI S.p.A., I-20152 Milano (IT)
(72) Inventor: Giovanardi, Renato, I-20090 Buccinasco (Milano) (IT); Lebano, Luigi, I-20064 Gorgonzola (Milano) (IT)
(74) Representative: Dragotti, Gianfranco

(57) **Abstract**

The bag supply system containing in bag (16) the next to be used dialysate, is connected by connector (32) to and via the hand operated valve (40) to the indwelling catheter (10). Closing valve (20) and opening valve (40) allows used dialysate to be discharged through Y connector (22). Valve (24) seals the outflow tract at the end of discharge. Opening valves (20 and 40) allows fresh dialysate to flow into the perithoneum. Once filling is completed, valve (30) can be activated, thus choking line (26) with pins (44) and forked members (46) and severing it with built-in sickle shaped blades (54) from the rest of the system at a location above the point of choking.

## Description

The present invention relates to ambulatory peritoneal dialysis (also known as C.A.P.D.) and in particular the periodic replacement by means of infusion of the spent dialysis solution (after remaining for a few hours inside the peritoneal cavity and subsequent discharging) with fresh dialysis liquid.

As is known, in peritoneal dialysis a bag containing dialysis liquid is connected up to the peritoneal cavity, usually by means of a peritoneal catheter implanted permanently in the patient's abdomen.

Filling of the peritoneal cavity may occur by means of gravity - in which case the bag is placed at a suitable height from the patient and the difference in height regulates the speed of infusion, or entry, of the fresh liquid into the peritoneal cavity - or by means of a mechanical pump of the membrane or peristaltic type.

As already mentioned, the fresh dialysis liquid remains inside the peritoneal cavity for a predetermined period of time, following which it is discharged and usually disposed off, then providing to the detachment, from the peritoneal catheter permanently implanted in the patient's abdomen, of the assembly consisting of the bag or bags which are now empty and the connecting pipes (an assembly which is also known as a "pipeline set"), closing temporarily (i.e. for the time occurring between one dialysis session and the next one) the inlet mouth of the peritoneal catheter.

This set of operations gives rise to considerable problems and drawbacks, the main one of which is the risk of bacterial contamination of the peritoneal cavity (peritonitis) during the attachment and in particular detachment of the pipeline set to/from the peritoneal catheter.

In an attempt to overcome this problem various solutions have been proposed, such as for example the use of disinfectant liquids both during handling and with specific reference to the portion or end part of the peritoneal catheter protruding from the patient's abdomen.

These, however, are complicated and cumbersome solutions which make even more difficult a treatment which in itself is already physically and psychologically demanding for the patient.

It must be added that even so it is not entirely excluded that, even for a very short time, a condition may occur where there is free communication between the peritoneal cavity and external environment.

The main object of the present invention is just that of solving the problems and drawbacks briefly mentioned above and in particular eliminating the risk of bacterial contamination, avoiding at the same time the need for using protective and/or preventive systems which are complicated and costly in addition to being not entirely effective.

A more specific object of the present invention is that of providing means suitable for preventing, at the end of the phase involving filling of the peritoneum with fresh dialysis liquid, bacterial contamination during detachment of the pipeline set from the peritoneal catheter.

A further object of the present invention is that of avoiding bacterial contamination during discharging of the used dialysis liquid from the peritoneum before the start of the supply phase.

These and other objects of the present invention are achieved with the device for supplying and discharging dialysis liquid into/from the peritoneal cavity of the patient, of the kind comprising a bag filled with fresh and sterile dialysis solution, a first connecting duct having a first end connected, preferably permanently namely integrally, to the discharge mouth of said bag, the other end of said first duct consisting of a bifurcation having a first branch for discharging used dialysis solution while the second branch of said bifurcation has at its end a junction for connection to the peritoneal catheter of the patient, a first flow shut-off valve being provided in said first duct, while a second shut-off valve is provided in said first branch of said bifurcation, characterized in that said second branch of said bifurcation has inserted in it a shut-off valve device combined with a cutting member designed to sever said second branch in a position comprised between said shut-off valve device and the starting point of said bifurcation and in that between said connecting junction of said second branch and the external inlet mouth of said peritoneal catheter there is provided a second duct having its ends which can be respectively connected to said end junction of said second branch and to the inlet mouth of said catheter, a third shut-off valve being provided in said second duct.

As will appear more clearly from the herebelow detailed description of a preferred embodiment of the invention, the connection of the full bag of fresh dialysis solution to the peritoneal catheter is performed before discharging of the used solution from the peritoneal cavity so that handling of the end of said second duct for connection to the pipeline set and in particular to the end union of said second branch is performed preferably while the peritoneal cavity is still full of used solution and in any case in a condition such that the peritoneal cavity is isolated by said third shut-off valve from the external environment, following which, in the preferred embodiment, discharging of used dialysis solution causes simultaneously washing of said second duct, in the part comprised between said third shut-off valve and said end now connected to the free end of said second branch, said solution thus passing into said second branch and being discharged externally via said first branch of said bifurcation.

Once the discharge phase has been completed it is sufficient to open said first shut-off valve, close said second shut-off valve and finally keep said third shut-off valve open, so that the fresh dialysis solution flows out from said bag through said first duct, said second branch of said bifurcation and said second duct, filling the peritoneal cavity by means of gravity.

Obviously it is entirely possible that a pump of the aforementioned kind may be inserted between said first duct and said bag, for transfer of the dialysis solution from the bag to the peritoneal cavity.

Once the phase of supplying of the fresh dialysis solution into the peritoneal cavity has been completed, said first and third shut-off valves are closed and said shut-off valve device is operated so that any flow through said second branch of said bifurcation of said first duct is interrupted and at the same time said second branch is severed upstream (with reference to the direction of flow from the bag towards the peritoneal cavity) of the valve device, so that the device itself (in the closed condition), together with said second duct remains connected to said catheter which is permanently sealed even during detachment of the pipeline set with the corresponding bag which is now empty.

All the advantages of the invention may be better understood from the following description with reference to the accompanying drawings which show a preferred embodiment of the invention, it being understood that said embodiment is in no way limiting and is merely provided by way of example. In the drawings:
Figure 1 is a schematic depiction of the device according to the present invention, shown in the operating condition;
Figures 2 and 3 are, respectively, side elevation and axially sectioned views of the shut-off valve device combined with a cutting member;
Figure 4 is an exploded and sectioned view of the valve device according to Figure 2;
Figures 5 and 6 are side views of the two halves of the device according to Figure 2;
Figure 7 is a plan view from above of the valve device viewed in the direction of the arrow VII shown in Figure 3; and

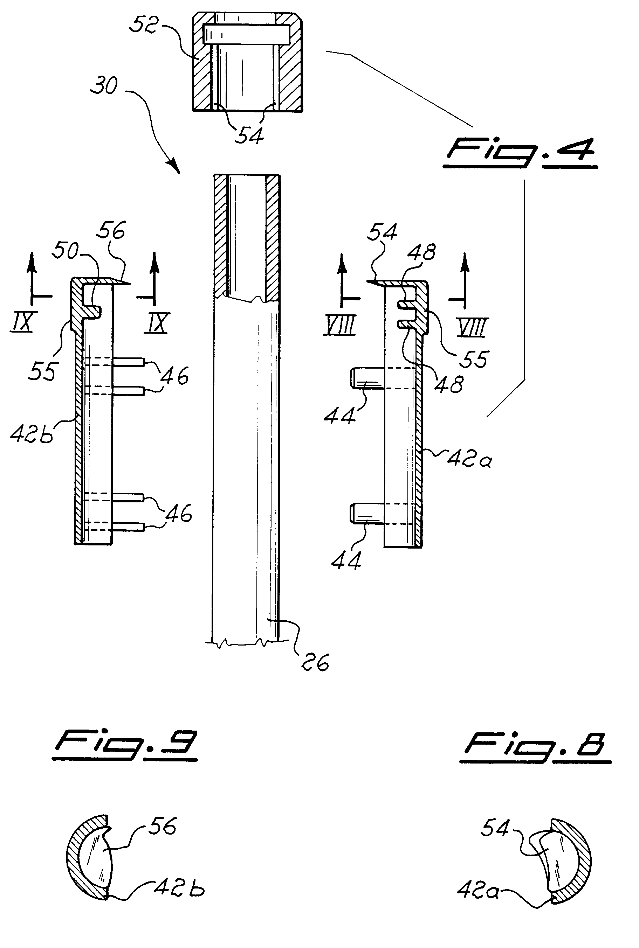

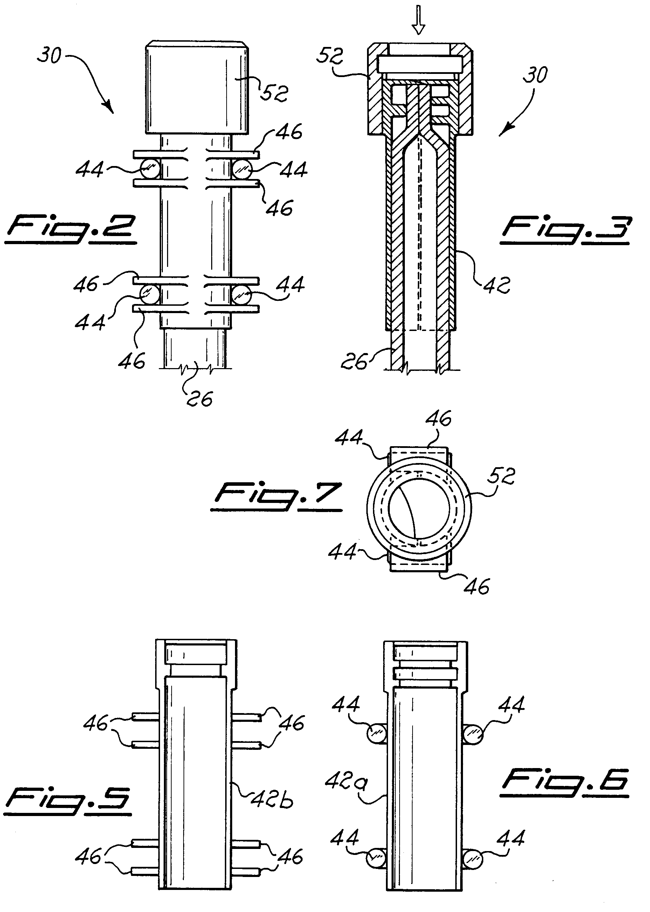

Figures 8 and 9 are views sectioned along the planes VIII-VIII and IX-IX of Figure 4.

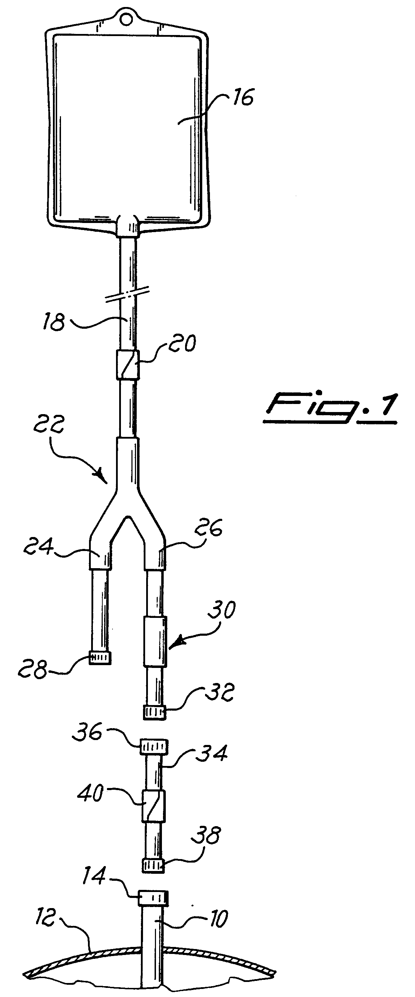

With reference first of all to Figure 1, the device according to the present invention is shown in a schematic and exploded condition with respect to the catheter 10 permanently implanted in the patient's peritoneum (represented by the abdominal epidermis denoted by the reference number 12, while the reference number 14 denotes a rapid-engagement junction of the universal kind (usually made of titanium).

The device according to the invention comprises a bag 16, normally with a capacity of two litres and having an outlet mouth preferably connected integrally to the upstream end of a first duct 18 provided with a shut-off valve 20.

This valve may also consist of a simple valve which externally constricts the walls of the duct 18, of the kind for example used for regulating the inflow speed in phleboclyses.

The other end of the tubular duct is formed by a bifurcation comprising a first branch 24 and a second branch 26.

The bifurcation 22 preferably consists of a three-way Y-junction, the stem of which is force-fitted onto the end of the first tubular duct 18.

The two branches 24 and 26 may also consist of tube sections coupled to the ends of the two branches of the aforementioned Y-piece, so as to provide the two branches with the desired length, simplifying at the same time the manufacture of the components of the device according to the invention.

The first branch 24 of the aforementioned bifurcation is provided with a shut-off valve (not shown) and terminates in an engagement junction 28 suitable for example for connection to a container for collecting or draining the used dialysis solution. In turn the second branch 26 of the bifurcation 22 has a shut-off valve device denoted generally by the reference number 30 which will be described in greater detail below with reference to the other figures, and terminates in an engagement or connecting junction 32, preferably of the snap-action kind.

A second duct 34 is located between the end of the second branch 26 of the bifurcation 22 and the external end of the catheter 10 provided with the engagement junction 14.

For this purpose the duct 34 is provided with a junction 36 designed to be connected to the junction 32 and with a junction 38 designed to be connected to the aforementioned end union 14 of the catheter 10.

The two ends of the duct 34 have inserted between them a flow shut-off valve 40 having the same features as the above mentioned valve 20.

Looking now at the other figures and firstly Figures 2, 3 and 4, the shut-off valve and cutting device 30 is shown inserted on the branch 26 of the bifurcation 22 with which the first duct 18 terminates.

This device comprises a substantially cylindrical casing 42, consisting of two halves 42a and 42b, obviously with a semi-circular cross-section, even though obviously the shape of the casing 42 may be different provided that it is compatible with the functions herebelow depicted.

For the snap-engagement of the two aforementioned halves, two pairs of pins 44 and two pairs of forked members 46 are provided, so that when the two halves are brought together until they coincide along their respective longitudinal edges (i.e. the edges parallel to their generatrices) the pins 44 snap-engage into their respective forked members 46.

In order to perform the function of closing and shutting off the flow through the duct 26, the half 42a of the casing 42 is provided with a pair of radial and parallel ribs 48, while the other half 42b is provided with a rib 50 also projecting radially towards the inside of the respective half-casing.

The external surface of the two casings 42a and 42b is provided with thickened portions 55 which operate as a cam surface during cooperation with the ribs 54 of the ring-nut 52.

When the ribs 48 and 50 are situated in radially opposite positions, they constrict the adjacent walls of the duct 26 in the manner depicted in Figure 3, this result being obtained by rotating the ring-nut or capsule 52 about the vertical or longitudinal axis of the device. The ring-nut 52, in fact, has a pair of vertical ribs 54 which act in the aforementioned manner when they are arranged in correspondence with the ribs 48 and 50.

As regards cutting of the duct 26, this operation is performed always with the rotation of the ring-nut 52, by means of two sickle-shaped blades 54 and 56 designed to cooperate when the two ribs 54 of the said ring-nut are in the aforementioned situation.

From the above description it is clear that simple rotation of the ring-nut 52 causes simultaneously constriction of the flow through the duct 26 and cutting of the latter above the constriction itself, namely in the downstream part of the duct 26 with reference to the direction of flow from the peritoneal catheter towards the bifurcation 22.

Summarising now the operation of the device according to the present invention, it comprises the following phases:
1) At the end of a dialysis-exchange phase the patient has the catheter 10 complete with the duct 34 (with the shut-off valve 40 closed), its outer end having connected to it the duct portion 26 terminating in the device 30 in the constricted condition.
   At this point the union 32 is detached from the union 36 and a new pipeline set is connected up in its place (said set comprising a bag 16 full of fresh dialysis liquid, duct 18 with the valve 20 closed and the bifurcation 22 having the first branch 22 with the respective shut-off valve closed and the branch 26 with the device 30 still integral).
   Opening of the valve 40 and of that present in the branch 24 allows discharging of the used dialysis solution from the peritoneal cavity.
2) After this discharge phase has been completed, the shut-off valve present in the branch 24 is closed and the the valve 20 is opened, so that the fresh dialysis solution starts to flow out from the bag 16 towards the peritoneal cavity.
3) Once the peritoneal cavity has been filled, the operator rotates the ring-nut 52, causing at the same time interruption of the flow between bag 16 and peritoneum and severing the duct 26 upstream of the constriction and hence the shut-off point formed in this way in the device 30.

From the above description it clearly appears that, with the device according to the present invention, the aims set by the invention are achieved in an advantageous manner.

Obviously it is clear that the ducts forming the device must be made of suitable materials, namely must be not only bio-compatible but also capable of being deformed (for the constricting action to be performed in the device 30) and of being cut. For this purpose the bifurcation 22 is provided in the form of a Y-shaped or three-way junction made of rigid and sturdy material, into the branches or arms of which portions of tube forming the ducts 18, 24 and 26 are force-fitted.

Moreover as regards the device 30, it has been described in the embodiment in which both the constriction of the duct 26 and cutting thereof are performed simultaneously.

It is obvious that these two operations may also be performed subsequentely and, in particular, by means of two different commands or operations, which means that the device 30 may be split into a valve for constricting the duct 26 and into a cutting device arranged upstream in the above specified direction.

## Claims

1. Device for supplying and discharging dialysis liquid for peritoneal dialysis, of the kind comprising a bag (16) filled with fresh and sterile dialysis solution, a first connecting duct (18) having a first end connected to the discharge mouth of said bag, the other end of said first duct consisting of a bifurcation (22) having a first branch (24) for discharging used dialysis solution while the second branch (26) of said bifurcation has at its end a junction (32) for connection to the peritoneal catheter (10) of the patient, a first flow shut-off valve (20) being provided in said first duct, while a second shut-off valve is provided in said first branch of said bifurcation, characterized in that said second branch (26) of said bifurcation has inserted in it a shut-off valve device (30) combined with a cutting member designed to sever said second branch (26) in a position comprised between said shut-off valve device (30) and the starting point of said bifurcation and in that between said connecting union (32) of said second branch (26) and the external mouth of said peritoneal catheter (10) there is provided a second duct (34) having its ends which can be respectively connected to said end union of said second branch and to the inlet mouth of said catheter, a third shut-off valve (40) being provided in said second duct (34).

2. Device according to Claim 1, characterized in that said shut-off valve and cutting device (30) comprises a substantially cylindrical casing (42) which has inside it a pair of radial and parallel ribs (48) and a radially projecting rib (50) designed to cooperate together so as to constrict the adjacent walls of the duct 26 in the shut-off condition, the device comprising moreover a ring-nut or capsule (52) provided with internal ribs (54) designed to engage with a cam action external thickened portions (55) of the casing (42), two sickle-shaped blades (54, 56) being connected to said ring-nut (52) and cooperating together in order to cut the duct (26) upstream of the constriction caused by engagement of the ribs (48 and 50) when the ring-nut (52) is rotated so as to engage the thickened portions (55) with the ribs (54).

3. Device according to Claim 2, characterized in that said substantially cylindrical casing (42) consists of two halves (42a, 42b) which can be snap-engaged together by means of two pairs of pins (44) and two pairs of forked members (46), said half (42a) being provided with said radial and parallel ribs (48) and said half (42b) being provided with said radial rib (50).

4. Device according to Claim 1, characterized in that said shut-off valve device (30) is arranged downstream, with respect to the direction of flow of the dialysis liquid from the bag to the peritoneal cavity of the patient, of a device known per se for cutting the said duct (26).
